(19) Europäisches Patentamt / European Patent Office / Office européen des brevets

(11) **EP 2 867 409 B1**

(12) **EUROPEAN PATENT SPECIFICATION**

(45) Date of publication and mention
of the grant of the patent:
**24.05.2017 Bulletin 2017/21**

(21) Application number: **13737685.1**

(22) Date of filing: **27.06.2013**

(51) Int Cl.:
*D21H 19/38* (2006.01)    *D21H 19/54* (2006.01)
*C08B 31/00* (2006.01)    *C08L 3/04* (2006.01)
*C09D 103/04* (2006.01)    *C12P 19/00* (2006.01)
*D21H 17/28* (2006.01)

(86) International application number:
**PCT/NL2013/050458**

(87) International publication number:
**WO 2014/003556 (03.01.2014 Gazette 2014/01)**

(54) **METHODS AND MEANS FOR COATING PAPER BY FILM COATING**

VERFAHREN UND MITTEL FÜR DIE BESCHICHTUNG VON PAPIER DURCH FILMBESCHICHTUNG

PROCÉDÉ ET APPAREIL DE COUCHAGE DE PAPIER PAR FILM COATING

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.06.2012 EP 12174461**

(43) Date of publication of application:
**06.05.2015 Bulletin 2015/19**

(73) Proprietor: **Coöperatie AVEBE U.A.**
**9641 GK Veendam (NL)**

(72) Inventors:
• **VAN DER MAAREL, Marc Jos Elise Cornelis**
**NL-9753 CC Haren (NL)**
• **TER VEER, Berend Cornelis Arend**
**NL-9471 PW Zuidlaren (NL)**
• **VRIELING-SMIT, Annet**
**NL-7741 PX Coevorden (NL)**
• **DELNOYE, Didier André Pierre**
**NL-9721 RW Groningen (NL)**

(74) Representative: **V.O.**
**P.O. Box 87930**
**2508 DH Den Haag (NL)**

(56) References cited:
**EP-A1- 0 690 170    EP-A1- 1 943 908**
**EP-A1- 2 172 489    WO-A1-00/66633**
**WO-A2-2008/082298    US-A1- 2010 058 953**

**Description**

This invention relates to the field of paper coating, more in particular to means and methods for providing paper with at least one layer of pigment using film coating to obtain a well printable surface. This invention also is directed to a coated paper comprising a paper and on the paper an amount paper coating composition of the present invention from which the water has been reduced or removed.

Surface characteristics of coated paper play a vital role in paper and print quality. Today, paper products must be designed with optimum surface properties to meet increasing performance requirements. Technological advances, including bigger and faster machines, require new, more cost-effective, and environmentally friendly coating methods and products.

The actual coating process usually comprises two steps: (i) the application of the coating medium containing pigments and binder (also referred to as "coating composition") onto the paper or board and (ii) the metering of the coating medium to the desired quantity (or coat weight). If the metering is done directly on the paper after application, the process is called direct coating or (puddle) blade coating. If, however, metering is done before the transfer of the coating medium to the paper or board, the process is called indirect coating or film coating. The present invention relates solely to means and methods for indirect / film coating.

In coating papers by film coating, a high solids content of the coating is beneficial (Glittenberg D., Filmpressenstreichen Was kann man von Spezialstärken erwarten. Wochenblatt für Papierfabrikation, 2009, (18-19), p.:856-862). The maximum solids of a coating composition is determined by the dry solids of the ingredients used to prepare the coating composition. The maximum solid content of the coating composition on the film coater is limited by the maximum solids in which the starch solution can be prepared, the stability of the starch solution and the rheology of the coating composition.

Immobilisation of the coating layer during application with the film coater is also important for the runnability. Immobilisation during the film coating process is influenced by the water retention capacity of the coating composition: on the one hand if the water retention is too low, the coating film will run dry during application; on the other hand if the water retention is too high the wet film layer will be too thick with misting as a result. This requires a balanced water retention of the coating composition, which is strongly determined by the binder system, especially starch binders. Starches known to the person skilled in the art for use in the preparation of coating compositions increase the water retention of the coating compositions, especially at high dry solids, thereby slowing down the immobilisation speed and creating more misting. At increasing speeds, misting increases significantly, especially when applying more than 50% starch as binder. Specially developed synthetic polymers have been added to reduce the misting tendency (Glittenberg D., Becker A., Voigt A., Kramm A., Van den Abbeele H. Elimination of Misting during high speed metered size press coating of starch containing coating colours with a new hyper-branched polymer, Professional paper making 2006, 2, p.:50-55). However, disadvantage of such polymers is the fact that they are oil based synthetics and add costs to the coating composition.

In order to obtain a high dry solids coating for film coating operation, a starch solution with high dry solids is required. The maximum solids of a starch solution is largely determined by the origin of the starch. In film coating application, partially depolymerised starches are being used as coating binder. Conventional depolymerisation methods for starch are oxidation, dextrination or enzymatic conversion with alpha-amylase. In an enzymatic conversion process, a starch slurry of native starch is degraded with alpha-amylase during the dissolution process. The maximum solids content is limited to 30-35% due to the slurry concentration and the high peak viscosity during dissolution. Moreover, starch solutions of enzymatically degraded starch can easily show retrogradation and therefore are less suitable for coating applications. Oxidised starches and dextrins are cook-up starches. These types of starches can be dissolved to 35%-45% maximum dry solids. In this way, coating compositions with higher dry solids can be achieved. Moreover, these types of starches are more visco-stable and are therefore generally applied in as binder in pigmented coating applications for the film coater. The maximum dry solids are determined by the starch slurry concentration in which the products can be suspended prior to dissolution. In general this is up to 40% for potato starch and up to 45% for cereal starches. However, these types of starches are dissolved in either a batch cooking or jet cooking process. Generally, live steam is used to cook the starch solutions which introduces condensation water which is unwanted because it reduces the maximum dry solids of the final starch solution with 3% to 4%. The amount of cook-up starch that can be used as binder is limited because a) the starch solution dilutes the coating composition too much, resulting in low dry solids and hence increased misting, and b) when the quantity of starch in the binder composition increases, runnability problems occur due to increasing dilitancy, resulting in too high coating weights and misting.

One possible way to circumvent the disadvantage of dilution is to use cold water soluble starches which can be added directly to the coating composition or which require a minimum of water to be dissolved. However, currently supplied cold water soluble starches are accompanied with several disadvantages. Commercially available low viscous cold water soluble starches such as malto-dextrins have too low binding power. Another range of cold water soluble starches are cold water soluble dextrins, such as yellow dextrins. However, these are accompanied with disadvantages in the film coating operation and paper properties: solubility in cold water is not 100%, the water retention is too high which gives misting and the yellow composition of the starch due to the dextrination process has a negative influence on the whiteness of the paper. A commercial example of a (partially) cold water soluble starch which may be used as coating binder in film coating application is C*Icoat, supplied by Cargill.

Therefore, there is a clear need for starches which can be used as binder in coating compositions for use in film coating, and which do not show above mentioned disadvantages, thus yielding coating compositions with excellent runnability, balanced water retention and, after application, excellent paper properties.

More specifically, the present inventors aimed at the provision of improved means and methods for film coating of paper using starch as the predominant binder. They particularly set out to identify a cold water soluble starch or a starch which can be added dry, or which requires a minimum amount of water to be dissolved, or which can be used as a liquid with dry solids content of 50% or higher, which can be used as binder in pigmented film coating formulations, while improving the runnability of the formulation.

Ideally, the starch product should have a viscosity in solution lower than 1000 mPas, preferably lower than 800 mPas measured with a Brookfield LVF at 50°C and 50% dry solids (w/w). Preferably, the starch can be used in combination with other binders such as latex.

Surprisingly, it was found that the objective of the invention can be achieved with a starch that has been gelatinized and then converted to highly branched starch using with a branching enzyme (E.C. 2.4.1.18) in combination with calcium carbonate as pigment.

Accordingly, in one embodiment the invention provides a method for preparing coated paper comprising the steps of:

a) providing a pigmented wet coating formulation comprising 2-20 parts of a binder and 100 parts of pigment, wherein at least 50% of the binder is highly branched starch (HBS) characterized by a molecular weight of between $0.5*10^5$ and $1*10^6$ Daltons and having a molecular branching degree of at least 6% and which is obtained by treatment of starch or a starch derivative with a glycogen branching enzyme (EC 2.4.1.18); and wherein at least 70% of the pigment is calcium carbonate; and
b) applying the pigmented wet coating formulation to paper by film coating, and drying the coated paper.

It was found that the unique combination of HBS as binder and calcium carbonate as pigment allows for the use of the starch derivative at a solids content higher than or equal to 50%, providing a stable solution with low viscosity and having sufficient binding power. The coating formulation has excellent runnability on the film coater while not showing film splitting nor misting.

Good results were obtained using about 10-20 parts of binder wherein HBS was combined with latex. In one embodiment, at least 50% of the total binder is HBS, preferably at least 55%, 60%, 65%, more preferably at least 70% of the binder is HBS, most preferably at least 80%, like at least 81, 83, 85, 86, 90%. In another aspect, HBS is the sole binder since starch is relatively cheap, more sustainable and renewable.

The degree of molecular branching as used herein refers to the relative amount of $\alpha$-1,6 glycosidic linkages over the total of $\alpha$-1,6 and $\alpha$-1,4 glycosidic linkages (($\alpha$-1,6/($\alpha$-1,6 +$\alpha$-1,4) *100%) and can be determined by methods known in the art, e.g. using a combination of reducing end determination/isoamylolysis (Palomo M et al. 2009 Appl. Environm. Microbiology, 75, 1355-1362; Thiemann, V. et al, 2006 Appl. Microb. and Biotechn. 72: 60-71) and measuring the total amount of carbohydrate present via the Anthrone/sulphuric acid method (see e.g. Fales, F. 1951 J. Biol. Chem. 193: 113-124).

EP 2172489 relates to methods and means for providing altered starch comprising treating starch with a branching enzyme of microbial origin, having improved resistance to retrogradation. However, it is completely silent about the use of microbially altered starch for the manufacturing of paper or as binder in paper coatings.

The use of branched starch in an aqueous coating liquid is disclosed in the art. EP0690170B2 describes a process for surface-sizing and/or coating paper. Example 2 of EP060170B2 describes a coating composition consisting of 100 parts by weight of SPS as pigment and 12.5 parts by weight of branched starch as sole binder in a coating formulation for direct coating using a puddle blade. This is distinct from the present invention describing the advantageous properties of HBS combined with calcium carbonate as pigment for use in indirect or film coating. As is shown herein below, a coating composition comprising the composition of Example 2 prepared according to EP060170B2 gives a poor runnability on the film coater.

WO2007/103517 discloses paper coating compositions which contain water, a binder, a high viscosity hydroxyethylcellulose polymer and a pigment, wherein the pigment comprises greater than about 80% by weight calcium carbonate pigment. Exemplary binders include those binders which are commonly used in the paper coating arts, e.g. protein, starch, styrene butadiene latex, styrene acrylate, polyvinyl acetate, and polyvinyl alcohol. WO2007/103517 does not teach or suggest using highly branched starch. The coating compositions of WO2007/103517 were specifically developed for use in blade coating methods, wherein high water retention values are preferred. It does not show or suggest that the coating compositions have good runnability on the film coater, showing good film splitting and low misting tendency. Moreover, WO2007/103517 does not define the binder system. In the examples, the coating colours are devoid of starch or contain less than 50% starch.

In step a) of a method according to the present invention, a pigmented wet coating formulation comprising 2-20 parts of a binder and 100 parts of pigment is provided, wherein at least 50% of the binder is HBS having a molecular branching degree of at least 6% and which is obtained by treatment of starch or starch derivatives with a glycogen branching enzyme.

Normal starch is composed of two constituents, the virtually linear amylose having alpha, 1-4 glycosidic linkages and the alpha, 1-6 branched amylopectin. There are also variants of starch with almost exclusively amylopectin (waxy) or containing a high amylose amount. BE are enzymes capable of converting the alpha, 1-4 glycosidic linkages present in amylopectin and amylose to alpha, 1-6 bonds, thereby creating new branch points. When incubated with gelatinized starch the amylose and/or long side chains of the amylopectin are transferred to amylopectin with the creation of new alpha, 1-6 glycosidic linkages. This results to a shortening of the average side chain length and a significant reduction of the interaction capacity of the branched molecules. The resulting starch derivative (herein referred to as HBS) has an average molecular weight (Mw) ranging between $0.5*10^5$ g/mol and $1*10^6$ g/mol, preferably between $0.8*10^5$ g/mol and $1.8*10^5$ g/mol, more preferably between $1*10^5$ g/mol and $1.6*10^5$ g/mol. The HBS typically has an average molecular weight (Mw) of about $1.2*10^5$ g/mol. Molecular mass can be determined using different techniques, known to the person skilled in the art. The molecular weight of the HBS of the invention was determined by GPC-MALLS-RI from Wyatt, USA equipped with a multiangle light scattering instrument (DAWN EOS) and an online viscometer (Viscostar). Refractive index was determined with RI2000 (Schambeck, Germany). The following set of columns was used: as guard column PwXL (Viscotek, USA) and as chromatography columns arranged in series: G4000PwXL and G5000PwXL (Viscotek, USA). A mixture of 50 mM $NaNO_3$ and 0.1 M NaCl and azide was used as running solution. The samples were solubilised in the buffer (mentioned above, 1 mg/ml) and filtered against 0.45 um before injection into the system. 0.2 ml was injected. Flow rate was 0.400 mL/min. Accuracy of the system was verified using dextrin standards (50K, 200K, 400K and 800K).

The HBS of the invention has no measurable reducing power/DE.

Moreover, the HBS for use in the present invention has no residual amylose content. The amylose content is suitably measured according to the $KI/I_2$ test. Solutions of starches that contain amylose will colour blue after addition of an aqueous mixture of KI and $I_2$ due to the formation of a complex between amylose and iodine. A solution of amylose free, amylopectin starch will colour purple although of lower intensity, due a different iodine-amylopectin complex. HBS starches from the invention, however, do not give any colouration upon addition of the $KI/I_2$ aqueous solution, showing the absence of any amylose or amylopectin like structures.

WO 00/66633 discloses branched glucose soluble polymers and a method for the production thereof. The branched starches from WO 00/66633 can be divided into two sub-families: one family characterized by molecular weights between $1*10^5$ and $1*10^6$ Daltons, between 2.5% and 5% branching and less than 1% reducing sugars, and a second family characterized by molecular weights between $1*10^7$ and $1*10^8$ Daltons, between 5% and 10% branching and more than 6% reducing sugars. In contrast, HBS for use in the present invention is characterized by molecular weights between $0.5*10^5$ and $1*10^6$ Daltons, at least 6% branching and typically contains less than 1% reducing sugars. WO 00/66633 furthermore fails to teach or suggest that branched starch can be used as binder in coating compositions.

US 2010/0058953 discloses a leguminous starch derivative for the preparation of a paper or folding carton coating composition. The starch derivative is obtained by dextrination, leading not only to new $\alpha$1-6 bonds but also to $\alpha$1-2 and $\alpha$1-3 branching. US 2010/0058953 mentions that compositions with this type of starch have a propensity for misting on a Film press, in particular at high speed, thus teaching away from the present invention. US 2010/0058953 does not define the binder system. In the examples, the binder system comprises a combination of latex and leguminous starch derivative starch. Starch makes up only 23% to 38% of the binder system. The leguminous starch derivative of US 2010/0058953 has an amylose content of less than 60%, preferably between 25 and 55%. In contrast, the HBS according to the invention is typically amylose free.

The combination of calcium carbonate pigment and HBS as disclosed in the present invention combines a number of already known functional properties of BE modified starch (low viscosity at high concentrations, no retrogradation and a transparent solution) with some unexpected functional properties (no film splitting, high binding power and balanced water retention) that are highly beneficial for use in coatings for film coating application e.g. as a (partial) latex substitute. In one aspect of the invention, HBS is the sole binder. Therefore, the invention also provides a film coating method wherein the coating formulation is devoid of latex.

As starting material for obtaining the abovementioned HBS any native/unmodified starch derived from non-GMO as well as GMO plant variants can be used, such as potato, corn, wheat, tapioca, waxy potato, waxy corn, waxy tapioca, high amylose potato, high amylose corn etc. In addition, modified starches are suitable including low DE maltodextrins or amylomaltase-treated starch (e.g. Etenia), and chemically modified starches.

In one embodiment, the starch derivative is selected from the group consisting of the products of the enzymatic, chemical and physical modifications of starch of any type. For example, the starch derivative is cationic starch, or oxidized starch, or phosphorylated starch.

The starch is first gelatinized before it can be brought into contact with the BE. The starch is gelatinized in a batch or continuous process in a steam injection device (jet cooker). The gelatinized starch is brought at the desired pH by the addition of acid or base and after the desired temperature has been reached the BE is added and the solution is kept at the desired temperature for a desired period of time. Thus, the HBS is obtained from starch or starch derivative in completely gelatinized form.

Alternatively, the BE is added to a starch suspension at room temperature and while mixing the slurry is heated to the desired temperature and kept at that temperature for the desired period of time. The conversion conditions and the amount of enzyme added vary widely depending on the starting material, the type of enzyme used and extent of the conversion. After the conversion has progressed to the desired extent, the enzyme can be inactivated by increasing the temperature or by lowering the pH of the incubation mixture. This can then be followed by a filtration and ion exchange step to remove protein. Subsequently, the pH is adjusted to the desired pH and starch mixture is subjected to drying e.g spray drying, or evaporation, or reverted osmosis, or another technique, to remove water and produce a high dry solid mixture.

The BE used can originate from any microbial source but preferably from a mesophilic or thermophilic microorganisms such as *Aquifex aeolicus* or *Rhodothermus obamensis.* Accordingly, in one embodiment the glycogen branching enzyme is a thermostable glycogen branching enzyme obtained from a mesophilic or thermophilic organism, preferably glycogen branching enzyme of *Aquifex aeolicus* or *Rhodothermus obamensis.*

Mixtures of HBS and other (starch-based) binders are also envisaged. Binders hold the pigment particles together, partially fill the voids between the pigment particles and also bond the coating to the base sheet or precoating. Binders may be either natural or synthetic materials. Examples of natural organic binders include starch and proteins such as soy protein or casein. Examples of synthetic binders include latices such as styrene-butadiene, vinyl acrylic copolymers, and polyvinyl acetate and water soluble polymers such as Polyvinylalcohol and CMC. Protein and casein binders have high binding strength and form relatively open coating structures. The limiting factor on use of protein binders is their high cost. They are normally used as cobinders in combination with latex. Styrene-butadiene (SBR) latices provide flexibility and hydrophobicity to the coating layer. Polyvinylalcohol is generally known as the binder with the highest binding power. However, price and dry solid content limit the amount that can be commercially and technically applied.

The invention is characterized in that HBS is used as binder together with calcium carbonate as the major pigment. Pigments are minerals which serve to provide optical and printability properties. White pigments provide opacity to cover the dark surface of the paperboard and brightness to obtain the desired degree of whiteness. High refractive index pigments such as titanium dioxide and calcined clay are very effective in scattering light and covering a dark surface of e.g. paperboard. Another key function of pigments is to control the degree of ink receptivity; this is dependent on the shape and size of pigment particles (morphology).

Preferably, at least 80% of the pigment is calcium carbonate, more preferably at least 90% or 95%. Calcium carbonate pigments are commercially available. Natural calcium carbonates are known generically as Ground Calcium Carbonate (GCC). In one embodiment, a method of the invention uses an ultrafine ground calcium carbonate, e.g. sold under the trademark Hydrocarb. In another embodiment, it is a Precipitated Calcium Carbonate (PCC) which is a synthetic calcium carbonate produced industrially by means of a recarbonisation process. Different grades of calcium carbonate pigments of different particle size distribution and optics can be used for coating applications. Preferably, at least 60%, like 70%, 75% or 80%, has a particle size of 2 $\mu$m or less. Suitable commercial calcium carbonate pigments include Hydrocarb 75 and Covercarb 75. Combinations of two or more types of calcium carbonates are also envisaged.

Suitable other pigments for use in combination with calcium carbonate include clay and titanium dioxide, for example Capim DG. Using the proper combination of pigments controls the mean pore structure and smoothness of the coated sheet surface. Some pigments, such as fine particle size clay, are capable of being aligned to provide uniform low angle reflectance of light upon calendering in an elastic nip; this is how gloss is developed. Other pigments provide an open coating structure which can be the key to providing bulking and coating porosity; examples of pigments with higher than average open area include calcium carbonate and calcined clay.

In step b) of a method of the invention, the pigmented wet coating formulation is applied to paper by film coating and the coated paper is dried. Film coating methods and film coating apparatuses are known in the art. In a film press, a film of the coating medium is formed and metered on a large diameter roll. This roll forms a nip with another roll. The paper or board web passes this nip and picks up a certain portion of the film. Application can be -but does not have to be - simultaneous on both sides. Metering is performed using metering rods, which can be either smooth or profiled. Profiled rods provide a certain volume of coating medium due to the open cross section in the profile. A deeper or coarser profile gives a higher coat weight than a fine profile. The application weight is mainly adjusted by choosing an adequate profile. Typical coating weights range from about 8-11 gsm/side. In one embodiment, the coating formulation is applied in a targeted amount of at least 6 gsm/side, preferably at least 7 gsm/side, more preferably at least 8 gsm/side, like 9, 10, 11 or 12 gsm/side.

Application may be done at any desired machine speed. As said, a coating formulation of the invention shows very little misting even at high speeds and high coat weights. Hence, machine speeds of at least 1100 m/min, preferably 1200 m/min or more preferable at least 1300 m/min may be used. For example, step b) comprises applying the coating formulation at a speed of at least 1200 m/min in an amount of at least 8 gsm/side.

Fine tuning of the application weight can be done by modifying the rod pressure. Profiled metering rods are mainly used for low viscosities (i. e. low solids contents of the coating medium) and low machine speeds. With increasing viscosity and speed, the rod loses contact with the roll due to the hydrodynamic forces of the coating medium, comparable to aquaplaning. Then, the application weight depends less on the profile and more and more on the hydrodynamic conditions, such as rod pressure, rod diameter, viscosity and speed. Consequently, a smooth metering rod is often used. Typical rod diameters are 12-38 mm for smooth rods. In one embodiment, a smooth rod having a diameter between about 20 and 30 mm is used. Larger diameters yield higher application weights. Higher viscosities and machine speeds require smaller rod diameters for the same application weight than lower ones. The coating medium is usually not transferred completely to the web. A certain amount remains on the roll and returns to the application unit where it is mixed with fresh coating medium. The transfer ratio depends on the acceptance behaviour of the web, on the properties of the coating medium and - to a limited degree - on the surface properties of the roll.

The pre-metered films usually have thicknesses of 7-20 $\mu$m (or ml/m$^2$). Lower values would require very high rod pressures and also coverage of the web would be insufficient. Higher values are not meaningful since the web has a limitation with respect to coating acceptance. If the amount of pre-metered coating medium is too high, the surface of the coated web appears uneven, with an "orange peel" character. At elevated machine speeds, the film split at the nip exit can create a fine mist of coating medium. This mist deposits on machine parts or even on the paper or board web. Since this misting increases with film thickness, it is the major limitation for the application weight at high speed. For typical applications using prior art coating compositions, misting becomes a limitation above 1500 m/min.

It has been observed by the industry that paper coatings utilizing greater than about 70% calcium carbonate by weight generally exhibits an undesirable rheological property of severe water loss from these coatings through absorption into the porous base sheet during application. This severe water loss is a phenomenon that may also be described as "poor water retention". This poor water retention property is very problematic for the application of paper coatings containing 100% GCC as a pigment, and even more severe of a problem with the use of PCC as a pigment in paper coating compositions. Coating compositions with poor water retention can run dry during film press operation due to strong absorption of the liquid coating medium by the paper web. This results in poor coverage and quality, and moreover to runnability problems due to web brakes. Because of this poor water retention property and associated runnability problems and in order to allow acceptable application of the wet coating to the porous base sheet, the coated paper industry has been forced to utilize a relatively low solids coating formulation, i.e. solids levels below 64% by weight, when 100% calcium carbonate is employed as a pigment. However, the drawbacks of the use of lower solids coating formulations creates two additional significant problems: 1) the use of lower solids coating translates into higher drying energy costs of the coated paper industry, and 2) lower solids of coatings generally produces lower overall quality coated paper, since during the drying step when more water is employed in the coating recipe, then more binder migration and coating shrinkage occurs thereby affecting the finished coated paper quality negatively.

On the other hand, coating compositions with high water retention are also associated with poor runnability on the film press. In case of strong water retention, the coating medium is not absorbed by the paper web in the nip of the film press, which results in a poor transfer ratio. The film split after the nip then causes severe misting, especially at increasing speeds and coat weights.

Due to its unique properties, the use of HBS as a binder as disclosed in the present invention allows to prepare wet coating formulations having a solids content of at least 60% by weight, preferably at least 65%, like 66%, 67% or 68%, more preferably at least 70% by weight. The unique coating formulation disclosed in the present invention allows to achieve coat weights above 7 g m$^{-2}$ without misting at high machine speeds, like 1800 m/min.

The paper web can be dried according to conventional methods. Infra Red (IR) drying and Air drying can be employed to dry the web after application of the liquid coating. Also drying cylinders may be applied to dry the coated paper. Usually, the paper is dried to a moisture content of 4-6% for finished paper, or to 2-3% when the paper is prepared for post treatment with one or more coating layers.

After the paper has been coated, it may be treated with a calendering step to increase the gloss and smoothness of the paper. The purpose of calendering is to increase the gloss of the paper and improve the printability. In a calendering device, the paper is run between two or more rollers. By compression and heating of the rollers, the paper is given a more smooth surface and unevenness of the surface is reduced to give a glossy appearance and improve printability. Usually two types of calenders are employed to treat coated papers: soft nip calender and multi nip calender. In a soft nip calender, the coated paper is compressed in 1 to 4 nips of which at least one roller is covered with an elastic material. In a multi-nip calender employs 6 to 12 rollers, which are either covered with an elastic material, or heated. Both the soft-nip calender as well as the multi-nip calender can be on-line or off-line.

The invention also relates to film coating formulations and methods for preparing the same. A paper coating composition of the present invention contains water, HBS as binder, and a pigment, wherein the pigment comprises greater than about 80% by weight calcium carbonate pigment. The pigment may comprise higher levels of calcium carbonate such as greater than about 85% by weight calcium carbonate pigment, preferably greater than about 90% by weight calcium carbonate pigment, more preferably greater than about 95% by weight calcium carbonate pigment. The calcium carbonate pigment may be in the form of a ground calcium carbonate or in the form of a precipitated calcium carbonate. The ground or precipitated calcium carbonate may comprise up to about 100% by weight of the pigment.

In one embodiment, a pigmented wet paper coating formulation comprises water, 2-20 parts of a binder and 100 parts of pigment, wherein at least 50% of the binder is highly branched starch (HBS) having a molecular branching degree of at least 6% and which is obtained by treatment of starch or starch derivatives with a glycogen branching enzyme (EC 2.4.1.18); and wherein at least 70% of the pigment is calcium carbonate.

In another embodiment, the pigmented paper coating composition has a dry solids content of at least 65% by weight, comprising at least 80% by weight calcium carbonate as its pigment component and a modified starch as binder, the modified starch being a highly branched starch (HBS) obtained by treatment of starch or starch derivatives with a glycogen branching enzyme (EC 2.4.1.18) and having a molecular branching degree of at least 6%.

Preferably, at least 70%, more preferably at least 80% of the binder is HBS. In one embodiment, HBS is the sole binder and/or calcium carbonate is the sole pigment.

Coating formulations are typically expressed in terms of dry parts, with the total dry parts of pigment in a given formulation always equal to 100. Other coating components are expressed in parts as a ratio to 100 parts pigment. Additionally, thickeners and rheology modifiers are applied to coating formulations to improve the runnability on machine. The type of thickeners or viscosifiers that can be applied are, but not limited to, water soluble cellulosic derivatives such as Carboxy Methyl Cellulose (CMC) and Hydroxy Ethyl Cellulose (HEC), naturally occurring gums such as guar gum and xanthan gum, of the type of alkali swellable emulsions (ASE), such as aqueous acrylic copolymer dispersions, of the type of hydrophobically modified alkali swellable emulsions (HASE). Thickeners are typically present in amounts ranging between 0.01 part and 1 part.

Other additives which can be present in the coating formulation of the invention include dispersing agents, defoamers, insolizbilisers or hardeners, dyes and optical brightening agents and / or biocides. These additives are present in the coating formulation in quantum satis.

For example, the coating composition comprises 100 parts pigment, 2 to 20 parts HBS, 0-10 parts of another type of modified starch, for example oxidized potato starch (Perfectacote), and 0 to 8 parts styrene-based emulsion polymer binder, wherein the pigment is calcium carbonate. In another example, the coating formulation contains 100 parts pigment, 5 to 20; or 5 to 15 parts; binder in which HBS comprises at least 50% of the total binder, preferably at least 60%, more preferably 70%, and wherein the pigment is calcium carbonate. A coating formulation is readily prepared by standard methods. Pigments are typically added as slurry with solids contents between 25% and 78%, and HBS generally as solution with solids content between 25% and 60%, preferably about 50%. The pH of the coating formulation can be adjusted, e.g. within the range of pH 8.0- 10.0 using a suitable base such as diluted NaOH. A coating formulation of the invention is characterized by a number of unique properties. First, it shows an excellent stability and very low viscosity at elevated temperatures (e.g. 50°C) when compared to other starch-based coatings, like those containing oxidised starch or cold water soluble dextrin. In one embodiment, the coating formulation has a Brookfield viscosity (RVDVII, 100 rpm, 25°C) between 100 and 2000 mPas, preferably between 200 and 1500 mPas, more preferably between 200 and 1200 mPas. The medium viscosity (Hercules High-shear 1000 rpm, 25°C) is typically below 400 mPas, preferably below 300 mPas, more preferably below 250 mPas. The capillary viscosity ($1*10$ exp6 /s) is preferably below 100, more preferably below 80, more preferably below 70 mPas.

Second, a coating formulation showed unexpectedly improved water retention behaviour. For film coating application, fast immobilisation of the coating composition in the nip is of importance to avoid film splitting and subsequent misting. To this end, coating compositions with fast immobilisation speeds values are preferred. Increasing the starch amount in coating compositions usually has the opposite effect: they decrease the immobilisation speed of the coating composition due to an increase of the water retention behaviour, as the carbohydrate is a strong water binder. Surprisingly however, a coating composition with HBS shows a much lower water retention compared to coating compositions with traditional coating starches and is therefore preferred for use in film application.

Also provided is a coated paper comprising a paper and on the paper an amount of paper coating composition according to the present invention from which the water has been reduced or removed. The coated paper is preferably obtainable or obtained by a method of the invention. The coated paper is characterized by an increased paper surface strength and/or increased paper brightness.

Still a further embodiment of the invention relates to the use of a coating formulation as disclosed herein in a process for film coating of paper or paperboard. Also provided is the use of a highly branched starch (HBS) having a molecular branching degree of at least 6% and which is obtained by treatment of starch or starch derivatives with a glycogen branching enzyme (EC 2.4.1.18) to improve the performance of a film coating process or a film coater apparatus.

As described herein above, improving performance comprises reducing misting, reducing water retention, increasing paper surface strength, increasing paper brightness, improving runnability, increasing immobilization speed, or any combination thereof.

The invention is further explained in and by the following Experimental Section

EXPERIMENTAL SECTION

**Materials and Methods**

The BE used was the product NS28067 of Novozymes, a pilot plant product containing BE of *Rhodothermus obamensis.*

The Highly Branched Starch (HBS) was produced by jet cooking a 17% dry solid potato starch slurry (145-153°C, 8 min residence time, pressure 4 bar) and after cooling down to 70°C and adjusting the pH to 6.1, 1000 units of enzyme (measured as the change in the absorbance of a iodine/iodide starch complex at 660 nm) were added per gram dry substance of starch. After 20h of incubation the enzyme was inactivated by lowering the pH to 2.5 with 4M HCl and after 35 min the pH was readjusted to 4.5. Then the solution was filtered over a filter with pore size of 2-4 micrometer, followed by ion-exchange (Aquadem E200, Kruger). Finally, the solution was dried by evaporation of the water first at 61°C and then at 200°C (temp out 82°C). The activity of the enzyme is determined by monitoring changes in the iodine/iodide/amylose complex as a result of the branching enzyme activity. A substrate solution is prepared by adding 10 mg Amylose type III (Sigma) to 0.5 ml 2 M NaOH, subsequently adding 1 ml ultra pure water and then adjusting the pH by adding 0.5 ml 2 M HCl and 7.8 ml phosphate buffer (pH 7.2). A iodine/iodide stock solution is prepared by adding 0.26 g $I_2$ and 2.6 g KI to 10 ml ultra pure water. To 100 microliter of this stock solution 50 microliter 2 M HCl is added and 26 ml ultra pure water (stop reagent). The activity of the enzyme is determined by mixing 50 microliter of appropriately diluted enzyme to 50 microliter of amylose substrate solution and incubating this for 30 min at 60°C. Then 2 ml of stop reagent is added and after mixing well the absorbance is measured at 660 nm (the absorbance should be between 0.15 and 0.3). The activity (U/mL) is calculated using the following formula:

$$U/ml = (OD_{reference} - OD_{sample}) \times 100\% \times dilution / (OD_{reference} - OD_{blank}) / 30\ min / 0.05\ ml$$

Analysis of degree of branching

The samples were suspended/solubilized in cold water in a closed tube. In order to reach complete gelatinization all samples were heated, under ontinuous mixing, at 100 °C for 60 minutes. After cooling, acetate buffer was added to set the pH to 3.8. The samples were debranched by overnight incubation with a high dosage of the enzyme isoamylase (from Pseudomonas sp.) at 35°C. Finally, for chain length distribution analysis the samples were stabilized by addition of 9 volumes of DMSO. For the degree of branching assay the total carbohydrate was determined by the anthrone method (gives total glucose) and a Luff-Schoorl titration (gives total reducing glucose end groups) was done. The quotient of the two values (Luff/anthrone x 100%).

Starch derivatives:

Perfectacote 35 (P35): low viscous oxidised potato starch from AVEBE
Perfectacote 45 (P45): low viscous oxidised potato starch from AVEBE
Perfectacote 55 (P55): medium viscous oxidised potato starch from AVEBE
C*Icote: cold water soluble starch from Cargill
HBS: Highly branched starch, prepared as described above

Pigments

Hydrocarb 75 is a ground calcium carbonate and was obtained from Omya AG and used as a slurry with dry solids content of 78%. Hydrocarb 60 was obtained from Omya AG and used as a slurry with dry solids content of 78.4%. Capim DG is a Kaolin obtained from Imerys and used as a slurry with dry solids content of 70.5%.

Latex

Following types of lattices were used to prepare different coating compositions: EOC L607 is a Styrene-Butadiene co-polymer latex from EOC (Belgium). Styronal D517 is a Styrene-Butadiene copolymer latex from BASF. Basonal 2119.02 is a Styrene-n-butyl acrylate copolymer latex from BASF.

Additives for coating

Sterocoll FS is a synthetic thickener from BASF.

Dissolution of starch in hot water

Starch is added in cold water in a tank, equipped with a suitable stirrer. The obtained starch slurry is then heated in a water bath with well-dispersed live steam to a temperature of 95 °C. This temperature was maintained for 20 minutes. The starch solution is stored at 50°C before use.

Cold water solubility

Starch is added to water of 20°C in a 1 L beaker to make 600 g total weight, while stirring with an 8-holes stirrer at 600 rpm. The stirring is continued for 30 minutes. A precisely known amount of the starch solution is sieved over 75 $\mu$m sieve. The residue from the sieve transferred quantitatively to an aluminum cup and dried in an oven at 105°C. From the dry weight, the amount of non-dissolving starch can be calculated as a percentage. The Cold water solubility is expressed as 100%-non-dissolving part%.

Brookfield viscosity of a starch solution

Starch viscosity is measured in a 300 mL glass beaker with a Brookfield type LVF at 60 rpm and 50°C using the appropriate spindle. The value is recorded when the viscosity is stable or after 60 s.

Colour of a starch solution with Helliger tester

For this test a Hellige Neo-Komparator is used with glass cuvettes of 13 mm length. Fill the cuvette with the starch solution. Mount the most suitable Gardner Colour Disc into the apparatus. Turn the disc until the colour in left part equals that of the right part. Read the colour number from the Gardner Colour disc.

Preparation of a coating formulation

Add the pigment slurry (1000 g dry) into a 2000 mL beaker and disperse with a dilutor. Add water if necessary. Add starch (dry if cold water soluble) or a starch solution while stirring. Add synthetic binders to the dispersion while stirring. Finally, add other additives to the coating formulation. Adjust the formulation to the required pH with diluted sodium hydroxide solution (16 w/w%). Adjust the temperature of the coating formulation au bain Marie. Dilute the coating composition with water to the required dry solids.

Coating formulation rheology.

Brookfield viscosity: Brookfield RVDVII, 100 rpm, 25°C. The coating is measured in the 500 mL beaker.
Medium viscosity: Hercules DV-10 High-shear at 100 and 1000 rpm, 25°C. Bob/cup method is used, using Bob type A.
High shear viscosity: ACAV A2 Ultra High Shear capilary viscometer at $1*10^6$ s$^{-1}$.

Water retention: ÅA-GWR

Cover a filter paper (Whatman Cat. No. 1442-070) with a polycarbonate membrane (5.0 $\mu$m, Ankersmit, art. Nr. 13068) and place a cylinder on top of it. Meter 8 mL of coating formulation into the cylinder and close the cylinder. Bring it to pressure of 1.5 bar with air for 15 seconds. Weigh the filter paper to record the weight increase and calculate it into g/m$^2$. The water retention is expressed as the amount of water that is pressed out of the liquid coating formulation. Hence, a lower value indicates a stronger water retention.

Dry solids

Dry solid content of coating formulations is measured with a CEM Labwave 9000 at 100% power.

Paper testing

All papers are conditioned at 23°C and 50% moisture for at least three days prior to paper testing.

Bending stiffness

Bending stiffness is measured according to ISO-2493, using test strips of 38 mm width, bending to an angle of 15°, with a free length of 10 mm. The maximum force of resistance is recorded and expressed in mN. Five samples in machine direction (MD) and five in cross direction (CD) are measured.

Dry pick resistance

Dry pick resistance is measured according to NEN-ISO-3783, using an IGT pick testes type AIC 2-5. The test is run with 8 $\mu$m oil thickness on 1 cm disks, using a middle viscous IGT oil at a speed of 5 m/s. The point where the pick resistance starts is multiplied with the viscosity of the oil (520) and expressed as VVP (viscosity velocity product).

Wet pick/Ink transfer

Ink transfer and wet pick resistance are measured using a Prüfbau printability tester. Paper samples (47 mm x 230 mm) are mounted on test strips. 200 $mm^3$ ink (Huber no. 408002) is applied to the rubber inking reel which is equally distributes the ink during 30 s. Then the ink is transferred during 15 s to an ink-roll. 15 $mm^3$ of off-set water (86% demi-water, 10% iso-propanol, 4% combimat.) is added to the moistening station. The ink-roll is mounted to the print station. The paper sample is first (partly) wetted with the off-set water and immediately after printed with the ink-roll at a speed of 1 m/s. The sample is dried at the air. The ink density of the moistened part (a) as well as of the non-moistened part (b) of the paper test strip is measured using a densitometer. This gives the Ink transfer (a/b*100%) or Wet pick (100%-Ink transfer).

**EXAMPLE 1: Stability of the coating formulation**

This Example shows the excellent stability at high concentrations of HBS in comparison with other coating starches. For stability and colour measurements, starches were dissolved in hot water. The cold water solubility was determined as described above.

The HBS solution at a solids content of 50% or 35% is very stable with a very low viscosity at 50°C when compared to the other components tested. Except for the HBS solution that was very clear, colourless and transparent, all solutions were yellowish to brown.

Table 1 Brookfield viscosity (mPas) in time at 50 °C

| Time (h) | HBS 50% | HBS 35% | CWS dextrin 35% | P35 35% | P55 35% |
|---|---|---|---|---|---|
| 0 | 488 | 42 | 830 | 195 | 620 |
| 1 | 610 | 44 | 1260 | 220 | 780 |
| 2 | 580 | 44 | 1340 | 220 | 790 |
| 4 | 620 | 45 | 1400 | 215 | 810 |
| 24 | 580 | 44 | 1300 | 270 | 1210 |
| Colour (1h) | <1 | <1 | 8 | 4 | 3 |
| Cold water Solubility | >99.9% | >99.9% | 50% | Cook up starch | Cook up starch |
| CWS = Cold Water Soluble dextrin (= C*Icoat from Cargill) | | | | | |

EXAMPLE 2: Rheology

This Example illustrates the excellent rheology characteristics that HBS gives to a coating composition for film coating application, as sole binder, but also in combination with SB latex. The example also shows that the coating composition as prepared according to Example 2 of EP060170B2 does not have favourable rheology characteristics for application in a film coater.
Coating compositions for film coating application were prepared using HBS and tested on rheology behaviour. At least 50% of the binder is comprised of starch.

Reference coating 1 (R1) is a coating composition comprising 100 parts calcium carbonate pigment, 7.5 parts Perfectacote 45 and 7 parts latex.
Reference coating 2 (R2) is a coating composition comprising 100 parts Kaolin pigment (SPS Clay) and 12.5 parts HBS, according to EP0690170B2.
Coating 1 (C1) of the invention is a coating composition comprising 100 parts calcium carbonate pigment, 7.5 parts HBS and 7 parts latex.
Coating 2 (C2) of the invention is coating composition comprising 100 parts calcium carbonate pigment and 14.5 parts HBS as the sole binder.

**Table 2**

| Coating composition | Solids content | R1 | R2 | C1 | C2 |
|---|---|---|---|---|---|
| Hydrocarb 75 | 78% | 100 | | 100 | 100 |
| SPS Clay | 68% | | 100 | | |
| Latex EOC L607 | 50% | 7 | | 7 | |
| Perfectacote 45 | 33% | 7.5 | | | |
| HBS | 50% | | 12.5 | 7.5 | 14.5 |
| **Analysis** | | | | | |
| Dry solids | [%] | 66 | 70.3 | 69 | 67 |
| pH | | 9.5 | 9.5 | 9.5 | 9.5 |
| Brookfield 100 rpm | mPas | 700 | 4680 | 360 | 408 |
| Hercules High shear 1000 rpm @25°C | mPas | 122 | >>* | 57 | 206 |
| ACAV visco. @$1*10^6$ s$^{-1}$, 25°C | mPas | 49 | >>* | 50 | 64 |
| AAGWR @ 1.5 bar, 15 sec | g/m$^2$ | 19 | 17.5 | 40 | 12 |
| *: The viscosity of the Reference coating composition R2 is too high for measuring the Rotational high shear viscosity at 1000 rpm or the capillary viscosity (ACAV) at 70.3% solids as mentioned in EP0690170B2. | | | | | |

Due to its low viscosity at high concentrations, HBS can be used to increase the dry solid content of a coating considerably. Compare R1 with C1, where an increase of 3% can be achieved without increasing the high shear reology. In combination with the water retention properties it gives to the coating composition, makes HBS suitable to use as starch binder in a coating composition in a film coater at high solid concentration. In contrast, the coating composition containing HBS according to Example 2 of EP0690170B2 is not suitable for film coating application.

Table 2 shows that the High shear viscosity of Reference coating R2 is so high that viscosity could not be measured with Rotational viscometer (Hercules) nor with Capillary viscometry (ACAV). On the other hand, the coating composition with only HBS as binder from the invention (C2) shows to have acceptable (ACAV <70 mPas) rheology properties for film coating application.

**EXAMPLE 2B: Improved water retention behaviour for film coating**

Table 3 Coating compositions for film coating application

| | d.s. | | | | |
|---|---|---|---|---|---|

(continued)

| | | | | | |
|---|---|---|---|---|---|
| Hydrocarb 75 | 78% | 100 | 100 | 100 | 100 |
| P35 | 35% | 8 | | | |
| P55 | 35% | | 8 | | |
| HBS | 35% | | | 8 | |
| C*Icoat | 35% | | | | 8 |
| Styronal DL517 | 50% | 8 | 8 | 8 | 8 |
| pH | | 9 | 9 | 9 | 9 |
| Dry solids | [%] | 66 | 66 | 66 | 66 |
| T | [°C] | 25 | 25 | 25 | 25 |
| Brkfld 100 rpm @ 25°C | [mPas] | 660 | 1240 | 100 | 208 |
| Water retention | [g/m$^2$] | 21 | 9 | 64 | 26 |

Table 3 shows that a coating composition with HBS shows a lower water retention (higher value) compared to other coating starches in coating compositions with the same starch content, dry solids and temperature, which is better for application in film press.

**EXAMPLE 3: Coated paper to evaluate binding power of HBS**

This example shows that HBS as binder can be applied at high dry solids in increasing HBS/Latex ratios, while maintaining the same surface strength. Various coating compositions were prepared (see Table 4). The coating composition was applied onto 80 gsm woodfree basepaper from Burgo Ardennes using the puddle type blade from the Dixon coater at a speed of 50 m/min. Approximately 10 gsm of coating was applied. Sterocoll FS was added in order to adjust the Brookfield viscosity if necessary. The paper was dried to a moisture content of 5%. Before paper testing the papers were conditioned at 23°C and 50% relative humidity. As is clear from Table 4, the formulations with HBS yielded a higher surface strength compared to a formulation with a regular low viscous starch (Perfectacote 35).

Table 4

| | d.s. | A | B | C | D | E | F |
|---|---|---|---|---|---|---|---|
| Hydrocarb 75 | 78% | 100 | 100 | 100 | 100 | 100 | 100 |
| Perfectacote 55 | 35% | 6 | | | | | |
| Perfectacote 35 | 35% | | 6 | | | | |
| HBS | 50% | | | 6 | 8 | 10 | 12 |
| Latex DL517 | 50% | 6 | 6 | 6 | 4 | 2 | 0 |
| Sterocoll FS | Asis | | | 0.3 | 0.25 | 0.2 | 0.2 |
| pH | | 9 | 9 | 9 | 9 | 9 | 9 |
| Water | q.s. | | | | | | |
| Dry solids | [%] | 65.1 | 68.3 | 68.0 | 67.9 | 68.1 | 68.1 |
| Brookfield 100 rpm | [mPas] | 740 | 503 | 800 | 680 | 520 | 540 |
| Coatweight | [gsm] | 9.7 | 9.9 | 9.8 | 9.8 | 9.8 | 9.9 |
| IGT dry pick | [VVP] | 610 | 503 | 551 | 526 | 540 | 529 |

**EXAMPLE 4: Use of coating formulations in film coating**

This example shows that the application of the coating compositions according to the invention (C1 and C2 from Example 2) via film coating operation onto a paper web results in higher dry solids, improved runnability and improved paper properties in comparison with the reference coating compositions R1 and R2.

Coating compositions R1, R2, C1, C2 (see Example 2) were applied onto 66 gsm fine base paper (Burgo Ardennes) using a pre-metered size film press coater (Optisizer) at a speed of 1300 m/min applying 13 gsm/side using 25 mm smooth rod. The paper was dried to 4% moisture. The paper samples were conditioned and tested at 23°C and 50% relative humidity. Table 5 shows the runnability as well as the quality of the single coated paper produced with the pre-metered film press coater.

Although R2 could be prepared at high solids, it had to be diluted to give acceptable runnability on the film coater. Therefore, the coating composition was diluted to 61.9% solids on machine until acceptable rod pressure was achieved and the targeted coat weight could be applied. However, due to the low solids, the coating gave severe misting.

**Table 5: Single coated paper by film coating**

| Coating composition | Dry solids | R1 | R2 | C1 | C2 |
|---|---|---|---|---|---|
| Hydrocarb 75 | 78 | 100 | | 100 | 100 |
| SPS Clay | 68% | | 100 | | |
| Latex EOC L607 | 50 | 7 | | 7 | |
| Perfectacote 45 | 33 | 7.5 | | | |
| HBS | 50 | | 12.5 | 7.5 | 14.5 |
| **Analysis** | | | | | |
| Dry solids | [%] | 66.4 | 61.9 | 69.4 | 67.4 |
| pH | | 9.4 | 9.3 | 9.2 | 9.3 |
| Brookfield 100 | mPas | 803 | 330 | 413 | 263 |
| Machine data | | | | | |
| Coat weight | Gsm | 26 | 26 | 26 | 26 |
| Speed | m/min | 1305 | 1305 | 1305 | 1305 |
| Rod pressure | Bar | **2.1** | **2.4** | **2.0** | **2.3** |
| **Misting (0-5)** | | **3** | **5** | **2** | **0** |
| Paper testing | | | | | |
| Bending MD | mN | 109 | 115 | 101 | 128 |
| Bending CD | mN | 57 | 56 | 49 | 62 |
| **IGT dry pick c.s.** | **VVP** | **1560** | **1460** | **1400** | **1660** |
| **IGT dry pick u.s.** | **VVP** | **1210** | **1300** | **1200** | **1470** |
| Prüfbau ink transf [%] | % | 100 | 87 | 98.9 | 89 |
| Wet pick [%] | % | 0 | 12 | 1.1 | 11 |

**EXAMPLE 5: Exemplary coating formulations**

Table 6 Coating formulations according to the invention

| | | 1 | 2 | 3 | 4 |
|---|---|---|---|---|---|
| Hydrocarb 60 | | 85 | | | |
| Hydrocarb 75 | | 15 | 70 | 80 | 90 |
| Capim DG | | | 30 | 20 | 10 |
| HBS | | 6 | 7 | 7 | 7 |
| Basonal 2119.02 | | 6 | | | |

(continued)

|  | 1 | 2 | 3 | 4 |
|---|---|---|---|---|
| Styronal DL 517 |  | 5 | 5 | 5 |
| Sterocoll SL | 0.15 |  |  |  |
| FinFix 5 | 0.15 |  |  |  |
| Dry solids (%) | 72 | 70.0 | 69.9 | 70.2 |
| Brkfld 100 rpm @ 25°C | 1070 | 330 | 236 | 220 |
| Hercules High shear 1000 rpm @25°C | 205 | 140 | 106 | 107 |
| Water retention | 56 | 69 | 75 | 76 |

**Claims**

1. A method for preparing coated paper comprising the steps of:

    a) providing a pigmented wet coating formulation comprising water, 2-20 parts of a binder and 100 parts of pigment, wherein at least 50% of the binder is highly branched starch (HBS) **characterized by** a molecular weight of between $0.5*10^5$ and $1*10^6$ Daltons and having a molecular branching degree of at least 6% and which is obtained by treatment of gelatinized starch or starch derivative with a glycogen branching enzyme (EC 2.4.1.18); and wherein at least 70% of the pigment is calcium carbonate; and
    b) applying the pigmented wet coating formulation to paper by film coating and drying the coated paper, preferably comprising applying the coating formulation at a speed of at least 1200 m/min in an amount of at least 7 gsm/side by film coating.

2. The method according to claim 1, wherein the HBS has a molecular weight between $0.8*10^5$ g/mol to $1.8*10^5$ g/mol, preferably between $1*10^5$ g/mol and $1.6*10^5$ g/mol.

3. The method according to claim 1 or 2, wherein the HBS does not contain any residual amylose.

4. The method according to any one of the preceding claims, wherein said starch or starch derivative is selected from native, unmodified and chemically modified starch derived from non-GMO as well as GMO plant variants, preferably wherein the starch or starch derivative is derived from potato, corn, wheat, tapioca, waxy potato, waxy corn, waxy tapioca, like high amylose potato, high amylose corn, and modified starches including low DE maltodextrins and amylomaltase-treated starch.

5. The method according to claim 4, wherein the starch derivatives are selected from the group consisting of the products of acid or enzymatic hydrolysis of starch and the products of the chemical and physical modifications of starch of any type, preferably wherein the starch derivative is cationic starch, oxidized starch or phosphated starch.

6. The method according to any one of the preceding claims, wherein said glycogen branching enzyme is a thermostable glycogen branching enzyme obtained from a mesophilic or thermophilic organism, preferably glycogen branching enzyme of *Aquifex aeolicus* or *Rhodothermus obamensis.*

7. The method according to any one of the preceding claims, wherein HBS is the sole binder.

8. The method according to any one of the preceding claims, wherein the pigmented wet coating formulation comprises 100 parts pigment, 2 to 20 parts HBS, 0-10 parts of another modified starch, and 0 to 8 parts styrene-based emulsion polymer binder, and wherein the pigment is calcium carbonate.

9. The method according to any one of the preceding claims, wherein the solids content of the wet coating formulation is at least 60% by weight, preferably at least 65%, more preferably at least 70% by weight.

10. A pigmented wet paper coating composition comprising water, 2-20 parts of a binder and 100 parts of pigment, wherein at least 50% of the binder is highly branched starch (HBS) **characterized by** a molecular weight of between

0.5*10$^5$ and 1*10$^6$ Daltons and having a molecular branching degree of at least 6% and which is obtained by treatment of starch or starch derivatives with a glycogen branching enzyme (EC 2.4.1.18); and wherein at least 70% of the pigment is calcium carbonate.

11. The coating composition according to claim 10, having a dry solids content of at least 65% by weight, comprising at least 80% by weight calcium carbonate as its pigment component and a modified starch as binder, the modified starch consisting of said highly branched starch (HBS).

12. The coating composition according to claim 10 or 11, wherein said HBS has a molecular weight between 0.8*10$^5$ g/mol to 1.8*10$^5$ g/mol, preferably between 1*10$^5$ g/mol and 1.6*10$^5$ g/mol.

13. Coating composition according to any one of claims 10 to 12, comprising 100 parts pigment, 2 to 20 parts HBS, 0-10 parts of another modified starch, and 0 to 8 parts styrene-based emulsion polymer binder, and wherein the pigment is calcium carbonate.

14. Coating composition according to any one of claims 10-13, comprising 100 parts pigment, 5 to 20 parts binder in which HBS comprises at least 50%, preferably at least 60%, more preferably 70%, of the total binder and in which said pigment is calcium carbonate.

15. A coated paper comprising a paper and on the paper an amount of paper coating composition according to any one of claims 10-14 from which the water has been reduced or removed.

16. Coating composition according to any one of claims 10-14 for use in film coating of paper or paperboard.

17. The use of a highly branched starch (HBS) **characterized by** a molecular weight of between 0.5*10$^5$ and 1*10$^6$ Daltons and having a molecular branching degree of at least 6% and which is obtained by treatment of starch or starch derivatives with a glycogen branching enzyme (EC 2.4.1.18) to improve the performance of a film coating process or a film coater apparatus, preferably wherein improving the performance comprises reducing misting, reducing water retention, increasing paper surface strength, increasing paper brightness, improving runnability, increasing immobilization speed, or any combination thereof.


**Patentansprüche**

1. Verfahren zur Herstellung von beschichtetem Papier, umfassend die Schritte von:

a) Bereitstellen einer pigmentierten Nassbeschichtungsformulierung, umfassend Wasser, 2-20 Teile eines Bindemittels und 100 Teile von Pigment, wobei mindestens 50 % des Bindemittels hochverzweigte Stärke (engl. highly branched starch, HBS) ist, **gekennzeichnet durch** ein Molekulargewicht von zwischen 0,5*10$^5$ und 1*10$^6$ Dalton und mit einem molekularen Verzweigungsgrad von mindestens 6 %, und welche **durch** Behandlung von gelatinierter Stärke oder Stärkederivat mit einem Glycogen-verzweigenden Enzym (EC 2.4.1.18) erhalten wird; und wobei mindestens 70 % des Pigments Calciumcarbonat ist; und

b) Anwenden der pigmentierten Nassbeschichtungsformulierung auf Papier **durch** Filmbeschichtung und Trocknen des beschichteten Papiers, vorzugsweise umfassend Anwenden der Beschichtungsformulierung bei einer Geschwindigkeit von mindestens 1200 m/Min. in einer Menge von mindestens 7 g/m$^2$/Seite **durch** Filmbeschichtung.

2. Verfahren nach Anspruch 1, wobei die HBS ein Molekulargewicht zwischen 0,8*10$^5$ g/mol bis zu 1,8*10$^5$ g/mol, bevorzugt zwischen 1*10$^5$ g/Mol und 1,6*10$^5$ g/mol, hat.

3. Verfahren nach Anspruch 1 oder 2, wobei die HBS keinerlei Restamylose enthält.

4. Verfahren nach einem der vorhergehenden Ansprüche, wobei die Stärke oder das Stärkederivat ausgewählt ist aus nativer, unmodifizierter und chemisch modifizierter Stärke, die von Nicht-GMO- sowie GMO-Pflanzenvarianten abgeleitet ist, bevorzugt wobei die Stärke oder das Stärkederivat von Kartoffel, Mais, Weizen, Tapioka, Wachskartoffel, Wachsmais, Wachstapioka, wie Hochamylosekartoffel, Hochamylosemais, und modifizierten Stärken, einschließlich Maltodextrinen mit niedrigem DE und mit Amylomaltase behandelter Stärke abgeleitet ist.

5. Verfahren nach Anspruch 4, wobei die Stärkederivate ausgewählt sind aus der Gruppe, bestehend aus den Produkten von saurer oder enzymatischer Hydrolyse von Stärke und den Produkten der chemischen und physikalischen Modifikationen von Stärke jeder Art, bevorzugt wobei das Stärkederivat kationische Stärke, oxidierte Stärke oder phosphatierte Stärke ist.

6. Verfahren nach einem der vorhergehenden Ansprüche, wobei das Glycogen-verzweigende Enzym ein thermostabiles Glycogen-verzweigendes Enzym ist, erhalten von einem mesophilen oder thermophilen Organismus, bevorzugt Glycogen-verzweigendes Enzym von *Aquifex aeolicus* oder *Rhodothermus obamensis.*

7. Verfahren nach einem der vorhergehenden Ansprüche, wobei HBS das einzige Bindemittel ist.

8. Verfahren nach einem der vorhergehenden Ansprüche, wobei die pigmentierte Nassbeschichtungsformulierung 100 Teile Pigment, 2 bis 20 Teile HBS, 0-10 Teile einer anderen modifizierten Stärke und 0 bis 8 Teile Emulsionspolymer-Bindemittel auf Styrolbasis umfasst, und wobei das Pigment Calciumcarbonat ist.

9. Verfahren nach einem der vorhergehenden Ansprüche, wobei der Feststoffgehalt der Nassbeschichtungsformulierung mindestens 60 Gew.-%, bevorzugt mindestens 65 Gew.-%, bevorzugter mindestens 70 Gew.-% beträgt.

10. Pigmentierte Nasspapierbeschichtungszusammensetzung, umfassend Wasser, 2-20 Teile eines Bindemittels und 100 Teile von Pigment, wobei mindestens 50 % des Bindemittels hochverzweigte Stärke (HBS) ist, **gekennzeichnet durch** ein Molekulargewicht von zwischen $0,5*10^5$ und $1*10^6$ Dalton und mit einem molekularen Verzweigungsgrad von mindestens 6 %, und welche **durch** Behandlung von Stärke oder Stärkederivaten mit einem Glycogen-verzweigenden Enzym (EC 2.4.1.18) erhalten wird; und wobei mindestens 70 % des Pigments Calciumcarbonat ist.

11. Beschichtungszusammensetzung nach Anspruch 10, mit einem Trockenfeststoffgehalt von mindestens 65 Gew.-%, umfassend mindestens 80 Gew.-% Calciumcarbonat als ihre Pigmentkomponente und eine modifizierte Stärke als Bindemittel, wobei die modifizierte Stärke aus der hochverzweigten Stärke (HBS) besteht.

12. Beschichtungszusammensetzung nach Anspruch 10 oder 11, wobei die HBS ein Molekulargewicht zwischen $0,8*10^5$ g/mol bis zu $1,8*10^5$ g/mol, bevorzugt zwischen $1*10^5$ g/mol und $1,6*10^5$ g/mol hat.

13. Beschichtungszusammensetzung nach einem der Ansprüche 10 bis 12, umfassend 100 Teile Pigment, 2 bis 20 Teile HBS, 0-10 Teile einer anderen modifizierten Stärke und 0 bis 8 Teile Emulsionspolymer-Bindemittel auf Styrolbasis, und wobei das Pigment Calciumcarbonat ist.

14. Beschichtungszusammensetzung nach einem der Ansprüche 10-13, umfassend 100 Teile Pigment, 5 bis 20 Teile Bindemittel, in dem HBS mindestens 50 %, bevorzugt mindestens 60 %, bevorzugter 70 % des gesamten Bindemittels umfasst und in der das Pigment Calciumcarbonat ist.

15. Beschichtetes Papier, umfassend ein Papier und auf dem Papier eine Menge einer Papierbeschichtungszusammensetzung nach einem der Ansprüche 10-14, von der das Wasser reduziert oder entfernt wurde.

16. Beschichtungszusammensetzung nach einem der Ansprüche 10-14 zur Verwendung in Filmbeschichtung von Papier oder Karton.

17. Verwendung einer hochverzweigten Stärke (HBS), **gekennzeichnet durch** ein Molekulargewicht von zwischen $0,5*10^5$ und $1*10^6$ Dalton und mit einem molekularen Verzweigungsgrad von mindestens 6 %, und das **durch** Behandlung von Stärke oder Stärkederivaten mit einem Glycogen-verzweigenden Enzym (EC 2.4.1.18) erhalten wird, um die Leistung eines Filmbeschichtungsprozesses oder einer Filmbeschichtervorrichtung zu verbessern, bevorzugt wobei Verbessern der Leistung Reduzieren von Beschlagen, Reduzieren von Wasserretention, Erhöhen von Papieroberflächenstärke, Erhöhen von Papierhelligkeit, Verbessern von Verdruckbarkeit, Erhöhen von Immobilisierungsgeschwindigkeit oder jede Kombination davon umfasst.

**Revendications**

1. Procédé de préparation de papier couché comprenant les étapes suivantes :

a) préparation d'une formulation pigmentée pour couchage à l'état humide comprenant de l'eau, de 2 à 20 parties de liant et 100 parties de pigment, dans laquelle au moins 50 % du liant est un amidon fortement ramifié (HBS) **caractérisé par** un poids moléculaire compris entre $0,5*10^5$ et $1*10^6$ Daltons et ayant un degré de ramification moléculaire d'au moins 6 % et qui est obtenu par traitement d'un amidon gélatinisé ou d'un dérivé d'amidon avec une enzyme de ramification du glycogène (EC. 2.4.1.18) ; et dans laquelle au moins 70 % du pigment est un carbonate de calcium ; et

b) application de la formulation pigmentée pour couchage à l'état humide au papier par couchage en film et séchage du papier couché, comprenant de préférence l'application de la formulation pour couchage à une vitesse d'au moins 1200 m/min en une quantité d'au moins 7 gsm/côté par couchage en film.

2. Procédé selon la revendication 1, dans lequel l'amidon HBS a un poids moléculaire compris entre $0,8*10^5$ g/mol et $1,8*10^5$ g/mol, de préférence entre $1*10^5$ g/mol et $1,6*10^5$ g/mol.

3. Procédé selon la revendication 1 ou 2, dans lequel l'amidon HBS ne contient aucun amylose résiduel.

4. Procédé selon l'une quelconque des revendications précédentes, dans lequel ledit amidon ou dérivé d'amidon est choisi parmi un amidon natif, non modifié et chimiquement modifié dérivé de variantes végétales de type non-OGM ou OGM, de préférence dans lequel l'amidon ou le dérivé d'amidon est dérivé de la pomme de terre, du maïs, du blé, du tapioca, de la pomme de terre cireuse, du maïs cireux, du tapioca cireux, comme la pomme de terre à teneur élevée en amylose, le maïs à teneur élevée en amylose, et les amidons modifiés comprenant les maltodextrines à basse teneur en DE et l'amidon traité avec une amylomaltase.

5. Procédé selon la revendication 4, dans lequel les dérivés d'amidon sont choisis dans le groupe constitué par les produits de l'hydrolyse acide ou enzymatique de l'amidon et les produits issus des modifications chimiques et physiques des amidons de tout type, de préférence dans lequel le dérivé d'amidon est l'amidon cationique, l'amidon oxydé ou l'amidon phosphaté.

6. Procédé selon l'une quelconque des revendications précédentes, dans lequel ladite enzyme de ramification du glycogène est une enzyme de ramification du glycogène thermostable obtenue à partir d'un organisme mésophile ou thermophile, de préférence une enzyme de ramification du glycogène *d'Aquifex aeolicus* ou de *Rhodothermus obamensis.*

7. Procédé selon l'une quelconque des revendications précédentes, dans lequel l'amidon HBS est le seul liant.

8. Procédé selon l'une quelconque des revendications précédentes, dans lequel la formulation pigmentée pour couchage à l'état humide comprend 100 parties de pigment, de 2 à 20 parties d'amidon HBS, de 0 à 10 parties d'un autre amidon modifié, et de 0 à 8 parties d'un liant polymère en émulsion à base de styrène, et dans lequel le pigment est un carbonate de calcium.

9. Procédé selon l'une quelconque des revendications précédentes, dans lequel la teneur en matières solides de la formulation pour couchage à l'état humide est d'au moins 60 % en poids, de préférence d'au moins 65 %, plus préférablement d'au moins 70 % en poids.

10. Composition pigmentée pour couchage de papier à l'état humide comprenant de l'eau, de 2 à 20 parties de liant et 100 parties de pigment, dans laquelle au moins 50 % du liant est un amidon fortement ramifié (HBS) **caractérisé par** un poids moléculaire compris entre $0,5*10^5$ et $1*10^6$ Daltons et ayant un degré de ramification moléculaire d'au moins 6 % et qui est obtenu par traitement d'un amidon ou d'un dérivé d'amidon avec une enzyme de ramification du glycogène (EC. 2.4.1.18) ; et dans laquelle au moins 70 % du pigment est un carbonate de calcium.

11. Composition pour couchage selon la revendication 10, ayant en teneur en matières solides sèches d'au moins 65 % en poids, comprenant au moins 80 % en poids d'un carbonate de calcium à titre de composant de pigment et un amidon modifié à titre de liant, l'amidon modifié consistant en ledit amidon fortement ramifié (HBS).

12. Composition pour couchage selon la revendication 10 ou 11, dans laquelle ledit amidon HBS a un poids moléculaire compris entre $0,8*10^5$ g/mol et $1,8*10^5$ g/mol, de préférence entre $1*10^5$ g/mol et $1,6*10^5$ g/mol.

13. Composition pour couchage selon l'une quelconque des revendications 10 à 12, comprenant 100 parties de pigment, de 2 à 20 parties d'amidon HBS, de 0 à 10 parties d'un autre amidon modifié, et de 0 à 8 parties d'un liant polymère en émulsion à base de styrène, et dans laquelle le pigment est un carbonate de calcium.

14. Composition pour couchage selon l'une quelconque des revendications 10 à 13, comprenant 100 parties de pigment, de 5 à 20 parties de liant dans lequel l'amidon HBS représente au moins 50 %, de préférence au moins 60 %, plus préférablement 70 % du liant total et dans laquelle ledit pigment est un carbonate de calcium.

15. Papier couché comprenant un papier et sur le papier une quantité de composition de couchage pour papier selon l'une quelconque des revendications 10 à 14 dont la teneur en eau a été réduite ou éliminée.

16. Composition pour couchage selon l'une quelconque des revendications 10 à 14 utilisable dans le couchage en film du papier ou du carton.

17. Utilisation d'un amidon fortement ramifié (HBS) **caractérisé par** un poids moléculaire compris entre $0,5*10^5$ et $1*10^6$ Daltons et ayant un degré de ramification moléculaire d'au moins 6 % et qui est obtenu par traitement d'un amidon ou de dérivés d'amidon avec une enzyme de ramification du glycogène (EC. 2.4.1.18) pour améliorer la performance d'un procédé de couchage en film ou d'un appareil de couchage en film, de préférence dans laquelle l'amélioration de la performance comprend la réduction de la brumisation, la réduction de la rétention d'eau, l'accroissement de la résistance de la surface du papier, l'accroissement de la luminosité du papier, l'amélioration du passage sur machine, l'accroissement de la vitesse d'immobilisation, ou toute combinaison de ceux-ci.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2172489 A
- EP 0690170 B2
- EP 060170 B2
- WO 2007103517 A
- WO 0066633 A
- US 20100058953 A

**Non-patent literature cited in the description**

- **GLITTENBERG D.** Filmpressenstreichen Was kann man von Spezialstärken erwarten. *Wochenblatt für Papierfabrikation,* 2009, vol. 18-19, 856-862
- **GLITTENBERG D. ; BECKER A. ; VOIGT A. ; KRAMM A. ; VAN DEN ABBEELE H.** Elimination of Misting during high speed metered size press coating of starch containing coating colours with a new hyper-branched polymer. *Professional paper making,* 2006, 50-55
- **PALOMO M et al.** *Appl. Environm. Microbiology,* 2009, vol. 75, 1355-1362
- **THIEMANN, V. et al.** *Appl. Microb. and Biotechn.,* 2006, vol. 72, 60-71
- **FALES, F.** *J. Biol. Chem.,* 1951, vol. 193, 113-124